(19) Europäisches Patentamt

European Patent Office

Office européen des brevets

(11) Veröffentlichungsnummer: **0 283 700 B1**

(12) **EUROPÄISCHE PATENTSCHRIFT**

(45) Veröffentlichungstag der Patentschrift: **21.04.93**

(21) Anmeldenummer: **88101980.6**

(22) Anmeldetag: **11.02.88**

(51) Int. Cl.⁵: **C12N 13/00**, A23L 3/32, C02F 1/48

(54) **Verfahren und Vorrichtung zur Behandlung von Stoffen und/oder Mikro-Organismen mit Elektroimpulsen.**

(30) Priorität: **18.03.87 DE 3708775**

(43) Veröffentlichungstag der Anmeldung:
**28.09.88 Patentblatt 88/39**

(45) Bekanntmachung des Hinweises auf die
Patenterteilung:
**21.04.93 Patentblatt 93/16**

(84) Benannte Vertragsstaaten:
**AT BE CH DE ES FR GB GR IT LI LU NL SE**

(56) Entgegenhaltungen:
**FR-A- 2 059 215
FR-A- 2 336 480
FR-A- 2 554 827
US-A- 3 095 359**

(73) Patentinhaber: **Doevenspeck, Heinz
Sigurdstrasse 1
W-4950 Minden(DE)**

(72) Erfinder: **Doevenspeck, Heinz
Sigurdstrasse 1
W-4950 Minden(DE)**

(74) Vertreter: **Möller, Friedrich, Dipl.-Ing. et al
Meissner, Bolte & Partner Patentanwälte Hollerallee 73
W-2800 Bremen 1 (DE)**

EP 0 283 700 B1

Rank Xerox (UK) Business Services
(3. 10/3.5x/3.0. 1)

## Beschreibung

Die erfindung betrifft ein Verfahren nach dem Oberbegriff des Patentanspruches 1 und eine Vorrichtung zur Durchführung des Verfahrens nach dem Oberbegriff des Patentanspruches 7.

Aus DE-PS 12 37 541 ist ein Elektroimpulsverfahren bekannt, das zur Behandlung organischer und anorganischer Stoffe in vielen Fällen geeignet ist. Besondere Anwendungsgebiete dieses Elektroimpulsverfahrens liegen in der Gewinnung einzelner Phasen aus dispersen Systemen, was vor allem wesentliche Bedeutung für die Aufspaltung von Lebensmitteln hat, beispielsweise bei der Zuckergewinnung, der Trennung der Phasen (Eiweiß, Fett, Wasser etc.) von Fleischprodukten usw. Weiterhin ist aus dieser Druckschrift bekannt, daß mit Elektroimpulsen Bakterien (Mikroorganismen) abgetötet werden können. Dies ist zum Beispiel für die Haltbarmachung von Lebensmitteln oder die Trinkwasser- bzw. Abwasseraufbereitung von großer Bedeutung. Auch ist es bekannt, daß chemische Reaktionen durch Elektroimpulse der dort beschriebenen Art ausgelöst werden können und auf diese Weise auch Molekülketten organischer und anorganischer Substanzen züchtbar sind.

Bezüglich der Wirkung auf Mikroorganismen beschreibt die (nachveröffentlichte) DE-OS 35 38 194 eine anregende Wirkung auf den Stoffwechsel der Mikroorganismen, die zum Beispiel bei der Produktion von Methangas sehr interessant ist.

Bei diesem Elektroimpulsverfahren setzt man die in einem Elektrolyt vorhandenen Stoffe/Mikroorganismen bzw. die selbst einen Elektrolyt bildenden Stoffe kurzen elektrischen Feldern aus, die aus der Entladung einer oder mehrerer Kondensatoren längs einer durch mindestens zwei Elektroden gebildeten Entladungsstrecke zustandekommen. Als wichtig wurde hierbei gefunden, daß die Elektroimpulse steile Anstiegsflanken haben, zu deren Herstellung sich beispielsweise eine Schaltung nach der DE-PS 12 33 958 eignet. Die Entladung des Kondensators erfolgt über einen gesteuerten Schalter, nämlich ein Thyratron. Besonders vorteilhaft ist hierbei, wenn gleichzeitig die zu behandelnden Stoffe umgewälzt werden, wie dies beispielsweise in der DE-OS 31 16 623 beschrieben ist.

Bisher wurde dies als besonders wichtig bzw. als der einzig wichtige Faktor beschrieben, daß die Elektroimpulse eine definierte Engergiedichte aufweisen, also die Maximalspannung einen ganz entscheidenden Einfluß auf die Wirksamkeit des Verfahrens habe. Eine Steigerung der Wirksamkeit wird darüber hinaus dadurch erzielt, daß die Elektroimpulse in aufeinanderfolgenden Impulsgruppen (ggf. unterschiedlicher Amplitude auf das Substrat einwirken, wie dies zum Beispiel in der DE-OS 34 13 583 beschrieben ist.

Alle die oben beschriebenen Systeme sind zwar durchaus funktionsfähig und mit Vorteil anwendbar, jedoch hat es sich im Dauerbetrieb gezeigt, daß bei kontinuierlicher Arbeitsweise immer wieder Änderungen im "Wirkungsgrad" der Anlagen bzw. der Verfahren auftreten.

Ausgehend vom oben genannten Stand der Technik ist es Aufgabe der vorliegenden Erfindung, Verfahren und Vorrichtung der eingangs genannten Art dahingehend weiterzubilden, daß im kontinuierlichen Betrieb eine Steigerung der Wirksamkeit der Elektroimpulse erzielbar ist.

Diese Aufgabe wird verfahrensmäßig dadurch gelöst, daß man einen Optimalwert der für die Entladung bestimmenden Soll-Zeitkonstante bestimmt, bei welchem die gewünschte Wirkung der Elektroimpulse auf die behandelten Stoffe eintritt bzw. bei welchem der entsprechende Parameter maximal ist, daß man die Kapazität und/oder Impedanz auf die Soll-Zeitkonstante einstellt, und daß man die Zeitkonstante unabhängig von der Impedanz des Systems konstant hält.

Überraschenderweise hat es sich gezeigt, daß die "Abklingkonstante" der Entladung einen wesentlichen Faktor bezüglich der Wirksamkeit des Verfahrens darstellt. Wenn nun im kontinuierlichen Betrieb die unvermeidbaren Änderungen in der Zusammensetzung des Substrats zu einer Änderung der Impedanz des Systems, also des Widerstands zwischen den Entlade-Elektroden, führen, so wird gemäß der vorliegenden Erfindung diese Zeitkonstante trotz der Impedanzänderung konstant gehalten.

Um die Zeitkonstante trotz Änderung der Impedanz konstant zu halten, ist es nun gemäß einer Ausführungsform der Erfindung möglich, die Spannungserzeugung über eine Stromquelle durchzuführen, deren Innenwiderstand sehr hoch ist gegen die Impedanz zwischen den Entlade-Elektroden. Da aber die Entladespannungen im kV-Bereich liegen, sind hierzu umfangreiche gerätetechnische Maßnahmen möglich, will man nicht einen einfachen Vorwiderstand mit den ihm innewohnenden Wirkungsgrad mindernden Eigenschaften verwenden. Gemäß einer bevorzugten Ausführungsform der Erfindung wird darum ein Regelvorgang durchgeführt, bei welchem die Kapazität des Speicherkondensators (bzw. der Speicherkondensatoren) auf einen Wert nachgeregelt wird, bei welchem die erwünschte Entladezeitkonstante unter Berücksichtigung der momentanen Impedanz des Systems eingestellt ist. Bei einer anderen bevorzugten Ausführungsform der Erfindung geschieht die Nachregelung der Zeitkonstanten über die Zuschaltung von Serienwiderständen. Selbstverständlich ist es auch möglich, beide Lösungen parallel bzw. gleichzeitig zu verwenden, wobei eine Grob-/Feinabstimmung über die Kondensatoren bzw. über die Widerstände erfolgen kann.

Vorzugsweise mißt man den zu maximierenden Parameter - zum Beispiel die Menge an erzeugtem Methangas pro Zeiteinheit oder das Verhältnis von abgetöteten zu nicht-abgetöteten Keimen - am Systemausgang und ändert die Zeitkonstante so lange ab, bis der Parameter seinen Maximalwert erreicht. Der dann vorliegende Wert ist für das momentan behandelte Substrat charakteristisch und wird als Soll-Zeitkonstante gespeichert und den nachfolgenden Abgleichvorgängen zugrundegelegt. Bei der Optimierung geht man vorteilhafterweise nach Selbstlernmethoden (trial and error) vor, wobei kleine Änderungen der Soll-Zeitkonstante häufiger versucht werden als große Änderungen (Zufallsverteilung).

Mit der so gewonnenen Zeitkonstante vergleicht man die bei einem aktuellen Impuls tatsächlich auftretende Zeitkonstante und bildet aus der Differenz einen Korrekturwert, anhand dessen die Kapazität des Kondensators und/oder des Vorwiderstandes verändert wird, bevor die nächste Kondensatorentladung erfolgt. Durch diese Arbeitsweise kann man die Kondensatoren bzw. die Widerstände im stromlosen Zustand schalten, was erheblich einfacher ist als eine kontinuierliche Regelung.

Um eine möglichst genaue Regelung zu erhalten, ist es von Vorteil, wenn man über eine bestimmte Anzahl von Impulsen den Mittelwert der momentanen Zeitkonstante bildet und diesen Mittelwert den weiteren Parameter-Ermittlungen zugrundelegt.

Vorrichtungsmäßig wird die Aufgabe dadurch gelöst, daß eine Abgleichvorrichtung vorgesehen ist, die mindestens während des Entladevorganges mit dem Entladungsstromkreis zum Abtasten des Entladungsverlaufes verbunden ist, die Mittel zum Vergleichen des Entladevorganges mit einem vorbestimmten Wert und Mittel zum Angleichen des Entladevorganges an den vorbestimmten Wert aufweist.

Bevorzugte Ausführungsformen der Erfindung ergeben sich aus den Unteransprüchen sowie der nachfolgenden Beschreibung von Ausführungsbeispielen, die anhand von Abbildungen näher erläutert werden. Hierbei zeigt

Fig. 1   eine schematisierte Darstellung eines Systems zur Durchführung des Elektroimpulsverfahrens,

Fig. 2   eine Schemaskizze einer ersten Ausführungsform einer Vorrichtung zur Erzeugung von Elektroimpulsen,

Fig. 3   eine Schaltung der in Fig. 2 angedeuteten Abgleichvorrichtung,

Fig. 4   ein Diagramm zur Erläuterung der Wirkungsweise der Vorrichtung nach Fig. 3,

Fig. 5   eine weitere bevorzugte Ausführungsform der Erfindung in Darstellung gemäß Fig. 2,

Fig. 6   eine Schaltung der in Fig. 5 gezeigten Abgleichvorrichtung,

Fig. 7   eine weitere bevorzugte Ausführungsform der Erfindung in Darstellung nach Fig. 2 und

Fig. 8   die Schaltung einer Abgleichvorrichtung, wie sie in der Schaltung nach Fig. 7 verwendbar ist.

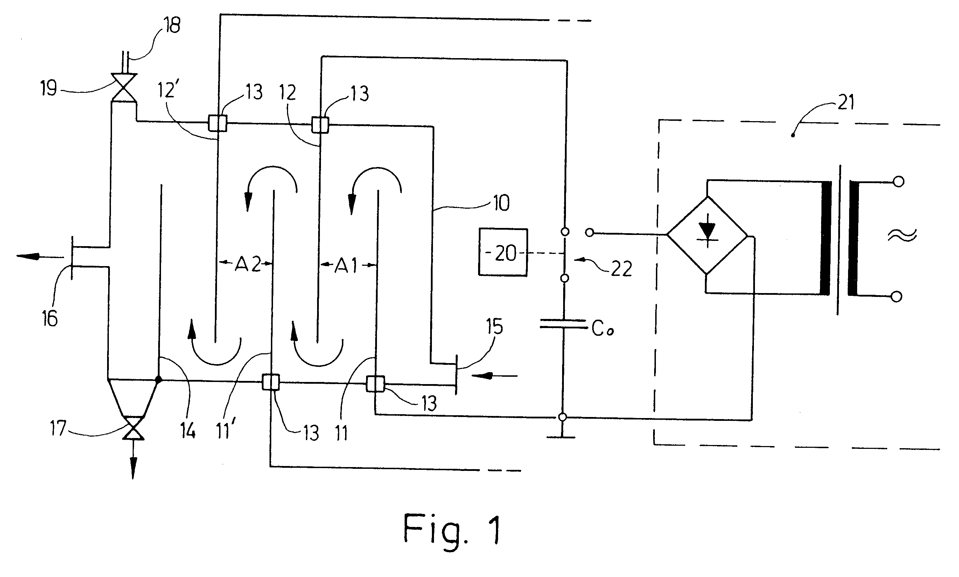

Bei dem in Fig. 1 gezeigten System ist ein Reaktor 10 vorgesehen, über dessen Einlaß 15 ein zu behandelnder Stoff, zum Beispiel Abwasser, in einem Labyrinth-Weg (siehe Pfeile) zwischen Elektroden 11, 12, 11', 12' hindurch zu einem Auslaß 16 gepumpt werden kann. In der Nähe des Auslasses 16 ist ein nach unten ragender Ablaß 17 angebracht. An der Decke des Reaktors 10 ist eine Gasleitung 18 mit einem Gasventil 19 vorgesehen. Die Elektroden 11, 12, 11', 12' sind über isolierende Durchführungen durch die Wand des Reaktors 10 in diesen hineingeführt.

Die Elektroden 11, 12 stehen über einen Umschalter 22 bzw. dessen erster Schaltstellung mit einem Kondensator $C_o$ inVerbindung. In der zweiten Schaltstellung des Umschalters 22 steht der Kondensator $C_o$ mit einem Netzteil 21 in Verbindung, über das er mit Gleichstrom auf eine definierte Spannung aufgeladen werden kann. Der Umschalter 22 wird über eine Impulssteuerung 20 gesteuert, welche diejenigen Zeitpunkte bestimmt, zu denen Impulse abgegeben werden. Der Umschalter 22 ist in Fig. 1 als mechanischer Schalter ausgebildet, wird jedoch in der Praxis vorteilhafterweise als elektrisch steuerbarer Schalter (Ignitron, Thyristor etc.) entsprechend der hier verwendeten hohen Spannungen ausgeführt. Bei der in Fig. 1 gezeigten Anlage ist nur ein einziger Kondensator vorgesehen, jedoch ist es von Vorteil, mehrere Elektrodenpaare in der Strecke mit verschiedenen Kondensatoren zu koppeln, um verschiedene Spannungen wirken zu lassen. Die Impedanz des Systems wird hierbei in erster Linie durch die Fläche der Elektroden 11, 12, deren Abstand A1 bzw. A2 zueinander sowie durch die Leitfähigkeit des durchgepumpten Mediums bestimmt. Die Leitfähigkeit hat hierbei im allgemeinen einen komplexen Wert.

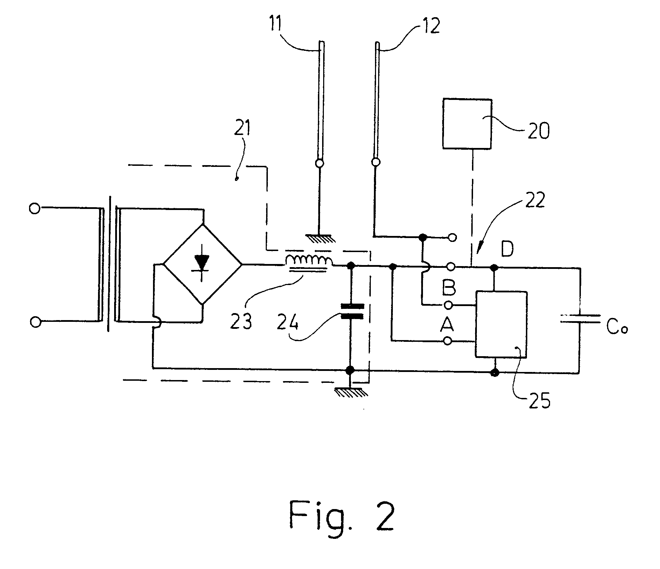

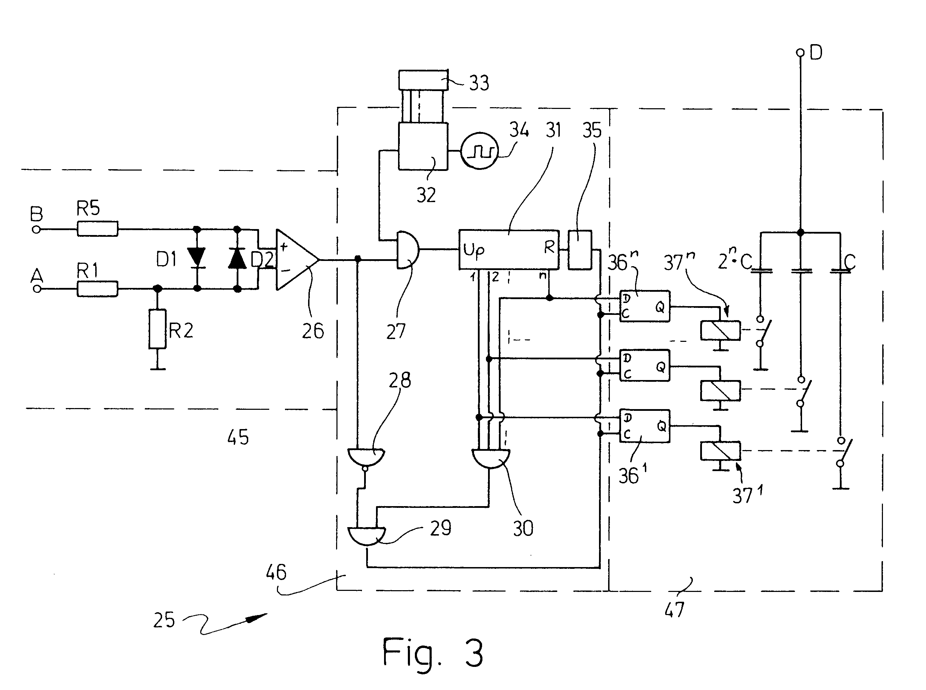

In Fig. 1 wurde der Entladekondensator $C_o$ als fester, unveränderbarer Kondensator dargestellt, die Impedanz des Systems ist ebenfalls nicht einstellbar. Gemäß der vorliegenden Erfindung wird jedoch anstelle des festen Kondensators eine Anordnung gewählt, wie sie in Fig. 2 beschrieben ist. Aus dieser Darstellung geht hervor, daß parallel zum Kondensator $C_o$ eine Abgleichvorrichtung 25 vorgesehen ist, deren einer Eingang A mit dem Ausgang des Netzteils 21 verbunden ist, das im vorliegenden Fall über eine Drossel 23 und einen Siebkondensator 24 stabilisiert ist, und dessen anderer Eingang mit der einen Elektrode 12 verbunden ist. Die Funktionsweise der Abgleichvorrichtung 25 wird im folgenden anhand von Fig. 3 in einer ersten Ausführungsform beschrieben.

Der Eingang A der Abgleichschaltung 25 führt auf einen Spannungsteiler $R_1$, $R_2$, dessen Teilungsverhältnis 1/e beträgt. Die geteilte Spannung ist auf den invertierenden Eingang eines Operationsverstärkers 26 geführt. Der zweite Eingang B der Abgleichschaltung 25 ist über einen Schutzwiderstand $R_5$ der aufgrund des Eingangswiderstandes des nachgeschalteten Operationsverstärkers 26 keinen spannungsändernden Einfluß aufweist, auf den nicht invertierenden Eingang des Operationsverstärkers 26 geschaltet. Zwischen den beiden Eingängen des Operationsverstärkers 26 sind Dioden $D_1$ und $D_2$ in einander entgegengesetzten Durchlaßrichtungen geschaltet, so daß die Spannungsdifferenz am Eingang des Operationsverstärkers 26 die Diodendurchlaßspannung nicht überschreiten kann. Hierbei sei bemerkt, daß vor den Eingängen A und B selbstverständlich eine Spannungsteilerschaltung (gleichen Teilungsverhältnisses für beide Eingänge) vorgesehen sein kann, damit die sehr hohen Kondensatorspannungen auf die üblichen niedrigen Pegel herabgesetzt werden können.

Der Ausgang der so gebildeten Komparatorschaltung 45, also der Ausgang des Operationsverstärkers 26 ist auf den Eingang einer Meßschaltung 46 geführt. Die Meßschaltung 46 umfaßt an ihrem Eingang ein UND-Gatter 27, dessen einer Eingang mit dem Ausgang des Operationsverstärkers 26 und dessen anderer Eingang über einen einstellbaren Teiler mit einem Taktoszillator 34 verbunden ist. Der Teiler 32 ist mit einem Sollwerteinsteller 33 verbunden, über den das Teilungsverhältnis des Teilers 32 eingestellt werden kann.

Der Ausgang des UND-Gatters 27 ist auf den Zähleingang Up eines Binärzählers 31 geführt. Die Zählausgänge 1 bis n des Zählers 31 sind auf die Eingänge eines ODER-Gatters 30 geführt, dessen Ausgang auf ein weiteres UND-Gatter 29 geführt ist. Ein zweiter Eingang des UND-Gatters 29 liegt über einen Inverter 28 auf dem Ausgang des Operationsverstärkers 26.

Der Ausgang des UND-Gatters 29 liegt auf dem Eingang eines Verzögerungsgliedes 35, das nach einem gewissen Zeitraum nach Anliegen eines positiven Spannungssprunges an seinem Ausgang einen Impuls abgibt. Dieser Impuls ist auf den Rücksetzeingang R des Zählers 31 geführt.

Die Zählausgänge 1 bis n des Binärzählers 31 sind weiterhin jeweils auf ein D-Flip-Flop $36_1$-$36_n$ geführt, deren C-Eingänge alle auf dem Ausgang des UND-Gatters 29 liegen.

Die Ausgänge Q der D-Flip-Flops sind auf die Erregerspulen von Relais $37_1$-$37_n$ geführt, welche Schließkontakte aufweisen, die einerseits mit (einer virtuellen) Masse, andererseits mit ersten Polen von Kondensatoren verbunden sind. Die anderen Pole der Kondensatoren sind zusammengeführt und als gemeinsame Anschlüsse D aus der Abgleichvorrichtung 25 herausgeführt. Bei Schließen der Relaiskontakte werden also die Kondensatoren parallel zum Kondensator $C_o$ geschaltet. Die Kapazitätswerte der Kondensatoren sind nun so ausgewählt, daß die niedrigste Stelle 1 des Zählers 31 einen Kondensator mit der Kapazität C parallel zum Kondensator $C_o$ schaltet, während die höchstwertige Stelle n des Zählers 31 einem Kondensator mit dem Wert $2^n$ x C entspricht.

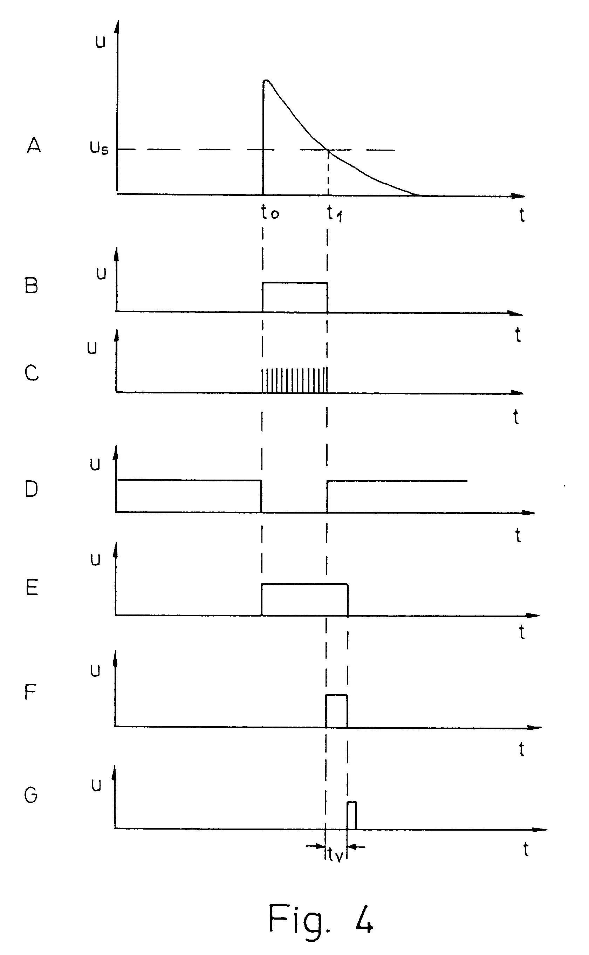

Im folgenden wird die Funktionsweise dieser Schaltung anhand der Abbildungen 4 näher erläutert.

In Abbildung 4A ist der Verlauf der Spannung U eines Entladeimpulses über die Zeit t aufgezeigt. Dieser Impulsverlauf liegt am Eingang B der Abgleichvorrichtung 25 an. Am Eingang A liegt die Ladespannung, also die Ausgangsspannung des Netzteils 21 an, so daß am invertierenden Eingang des Operationsverstärkers 26 eine Schwellenspannung $U_s$ liegt, welche entsprechend dem Teilungsverhältnis dem 1/e-Wert der Maximalspannung am Kondensator $C_o$ entspricht.

Wenn zum Zeitpunkt $t_0$ die Spannung U an der Elektrode 12 sprunghaft ansteigt, so springt der Ausgang des Operationsverstärkers 26 auf seine maximale (positive) Ausgangsspannung, wie dies in Fig. 4B gezeigt ist. Sobald die Spannung an der Elektrode 12 die Schwelle $U_s$ unterschreitet (Zeitpunkt $t_1$), fällt der Ausgang am Operationsverstärker 26 auf seinen Null-Wert bzw. seinen negativen Wert. Solange am Ausgang des Operationsverstärkers 26 eine positive Ausgangsspannung erscheint, läßt das UND-Gatter 27 die vom Taktoszillator 34 erzeugten und vom Teiler 32 heruntergeteilten Impulse in den Zähleingang des Zählers 31. Dies ist in Fig. 4C gezeigt.

Während der Zähler 31 hochzählt, steht am Ausgang des Invertierers 28 ein Null-Pegel an, so daß das UND-Gatter 29 sperrt, wie dies in Fig. 4D gezeigt ist.

Sobald der Zähler 31 den ersten Impuls gezählt hat, springt der Ausgang des ODER-Gatters 30 auf einen positiven Wert, wie dies in Fig. 4E gezeigt ist.

Wenn zum Zeitpunkt $t_1$ die Spannung an der Elektrode 12 die Schwelle $U_s$ unterschreitet, so springt der Ausgang des UND-Gatters 29 auf einen positiven Wert, da zu diesem Zeitpunkt der Ausgang des Inverters 28 auf einen positiven Wert springt und der Ausgang des ODER-Gatters 30 noch auf einem positiven Wert liegt. Dieser positive Ausgangswert, der in Fig. 4F gezeigt ist, gelangt einerseits auf die C-Eingänge der D-Flip-Flops $36_1$-$36_n$, so daß diese über ihre D-Eingänge die an den Ausgängen 1 bis n des Zählers 31 anstehenden Werte übernehmen. Gleichzeitig wird mit dem Spannungssprung des UND-Gatters 29 die Verzögerungsschaltung 35 getriggert, die nach einer Verzögerungszeit $t_v$ einenAusgangspuls definierter Länge abgibt, wie dies in Fig. 4G gezeigt ist. Der Ausgangspuls der Verzögerungsschaltung 35 setzt den Zähler 31 zurück, so daß der nächste Meßvorgang beginnen kann.

Die in die D-Flip-Flops $36_1$-$36_n$ übernommenen Ausgangswerte des Zählers 31 führen dazu, daß die Kondensatoren entsprechend dem gezählten Wert zu- oder fortgeschaltet werden. Es wird also mit dieser Schaltung direkt die Zeitkonstante gemessen und in einen zeit-proportionalen Kapazitätswert "übersetzt". Hierbei ist der Kondensator $C_o$ so ausgelegt, daß die durch ihn definierte Zeitkonstante kleiner ist als die gewünschte Zeitkonstante. Wenn nun beim Inbetriebnehmen der Anlage der erste Spannungspuls gemessen wird, so läuft eine, über den Teiler 32 definierbare Anzahl von Zählimpulsen in den Zähler 31, so daß nach Ende des ersten Spannungspulses einige Kondensatoren parallel zum Kondensator $C_o$ geschaltet sind. Wenn dieser Abgleich zum richtigen Ergebnis geführt hat, so wird beim nächsten Spannungspuls im Zähler 31 dieselbe Anzahl von Impulsen auflaufen, so daß die Schaltstellung der Relais $37_1$-$37_n$ nicht verändert wird. War der Abgleich noch nicht korrekt, so wird ein veränderter Wert übernommen, was schließlich zu einer Annäherung an den gewünschten Wert führt. Der gewünschte Wert ist hierbei über das Teilverhältnis des Teilers 32 bzw. über die Sollwerteinsteller 33 voreinstellbar.

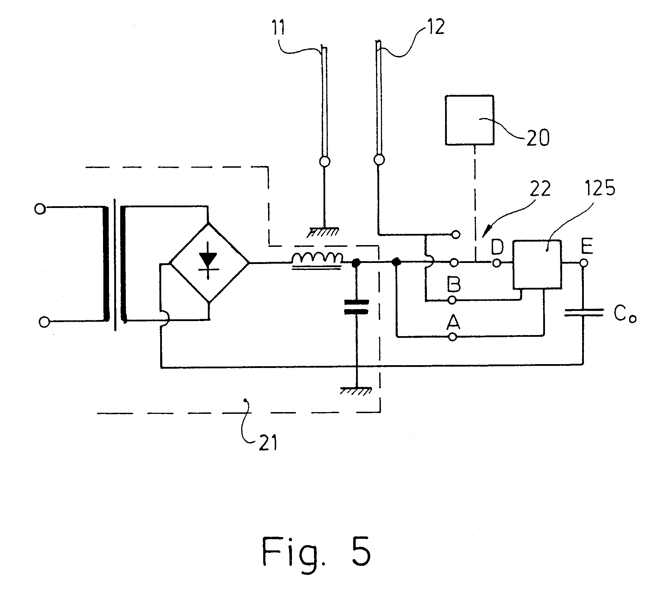

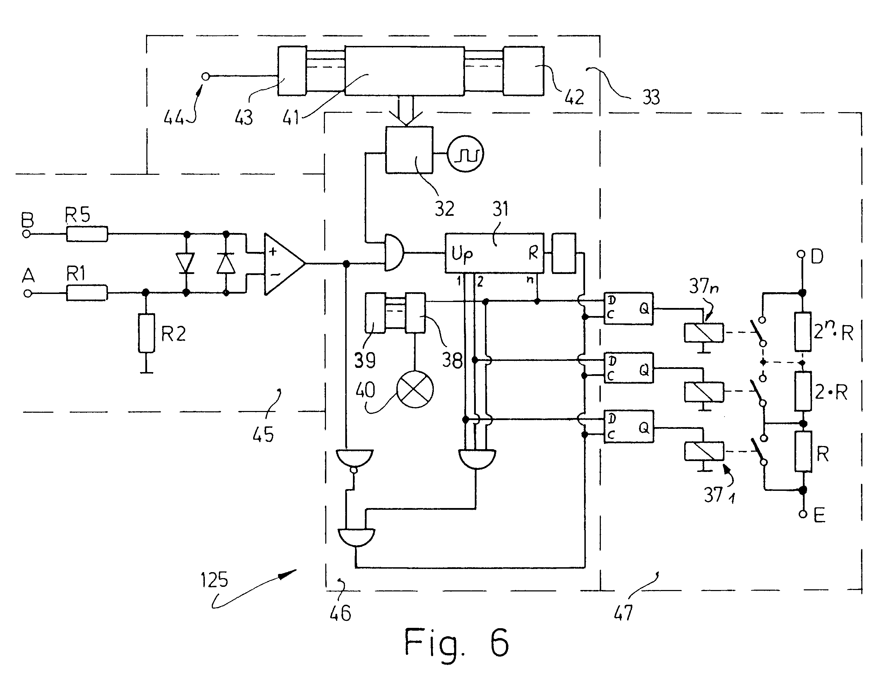

Bei der im folgenden anhand der Fig. 5 und 6 beschriebenen Ausführungsform der Erfindung ist die Abgleichvorrichtung 125 in Reihe zum Kondensator $C_o$ geschaltet. Bei dieser Abgleichvorrichtung ist die Kompensationsschaltung 47 als Reihenschaltung von Widerständen R, 2 x R, $2^n$ x R ausgebildet. Diese Widerstände können über die Relais $37_1$-$37_n$ in die Reihenschaltung einbezogen oder kurzgeschlossen werden. Je nach Widerstandsänderung ändert sich somit die Abklingkonstante des Entladungskreises.

Die Meßschaltung 46 ist bei dieser bevorzugten Ausführungsform der Erfindung gegenüber der vorher beschriebenen Form dadurch abgeändert, daß die Ausgänge 1 bis n des Zählers 31 zusätzlich auf einen Digital-Komparator 38 geführt sind, dessen andere Eingänge zu einem Grenzwerteinsteller 39 geführt sind. Der Komparator 38 vergleicht die im Zähler 31 gezählten Werte mit dem im Grenzwerteinsteller 39 eingestellten Wert und gibt dann ein Signal an eine Warnlampe 40, wenn der eingestellte Wert überschritten wird. Durch diese Anordnung kann man dann ein Warnsignal abgeben, wenn die Kompensationsschaltung 47 zum Beispiel an ihrer Grenze angelangt ist, also alle Widerstände in Reihe liegen oder alle Widerstände kurzgeschaltet sind. Alle Widerstände sind zum Beispiel dann in Reihe geschaltet, wenn zwischen den Elektroden 11, 12 ein Kurzschluß auftritt, während alle Widerstände kurzgeschlossen sind, wenn zwischen den Elektroden 11, 12 der Widerstand einen sehr hohen Wert annimmt, was beispielsweise beim Leerlaufen des Systems der Fall ist. Somit ist mit dieser bevorzugten Ausführungsform der Erfindung eine Warnung bei Fehlern im Gesamtsystem möglich.

Weiterhin wird bei der in Fig. 6 beschriebenen bevorzugten Ausführungsform der Erfindung der Teiler 32 über einen Mikroprozessor 41 eingestellt, der über eine Eingabeeinheit 42 programmierbar ist. An einem Eingang des Mikroprozessors 41 liegen die Ausgänge eines Meßwandlers 43, der die Ausgangssignale eines Meßfühlers 44 in Digitalworte wandelt. Der Fühler 44 ist so ausgebildet und im Reaktor 10 angebracht, daß er denjenigen Parameter mißt, den es zu optimieren gilt. Wenn beispielsweise der Reaktor 10 zur Erzeugung von Methan (Biogas) verwendet wird, so kann der Fühler 44 ein Durchflußfühler sein, der den in der Gasleitung 18 fließenden Volumenstrom mißt. Der Optimierungsvorgang läuft derart ab, daß der Mikroprozessor 41 zunächst einen Meßwert des Fühlers 44 speichert und dann den Zähler 32 auf einen zufällig gewählten Wert einstellt. Nach einer gewissen Zeitspanne, die durch die Arbeitsgeschwindigkeit des Systems bestimmt ist, tastet der Mikroprozessor 41 wieder den am Meßfühler 44 liegenden Meßwert ab und vergleicht diesen mit dem zuvor gewonnenen Meßwert. Hat sich der neue Meßwert gegenüber dem vorherigen Meßwert im Sinne einer Optimierung geändert, so wird beim nächsten Abänderungsversuch für das Teilerverhältnis der neue Meßwert als Grundlage beibehalten, die (geringfügig) geändert wird. War der zuerst gewonnene Meßwert "besser", so geht der Mikroprozessor 41 von diesem ersten Meßwert aus. Auf diese Weise ist eine Optimierung erzielbar, die dann besonders schnell (in wenigen Schritten) verläuft, wenn die zufällig gewählten Werte entsprechend einer Gauss-Verteilung um den bisher gefundenen Optimalwert verteilt sind, und zwar dahingehend, daß kleine Änderungen häufiger sind als große Änderungen (Evolutionsprinzip).

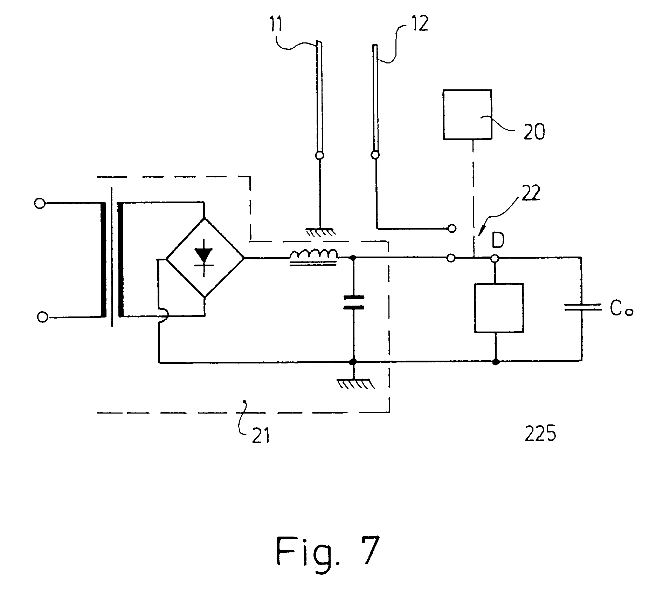

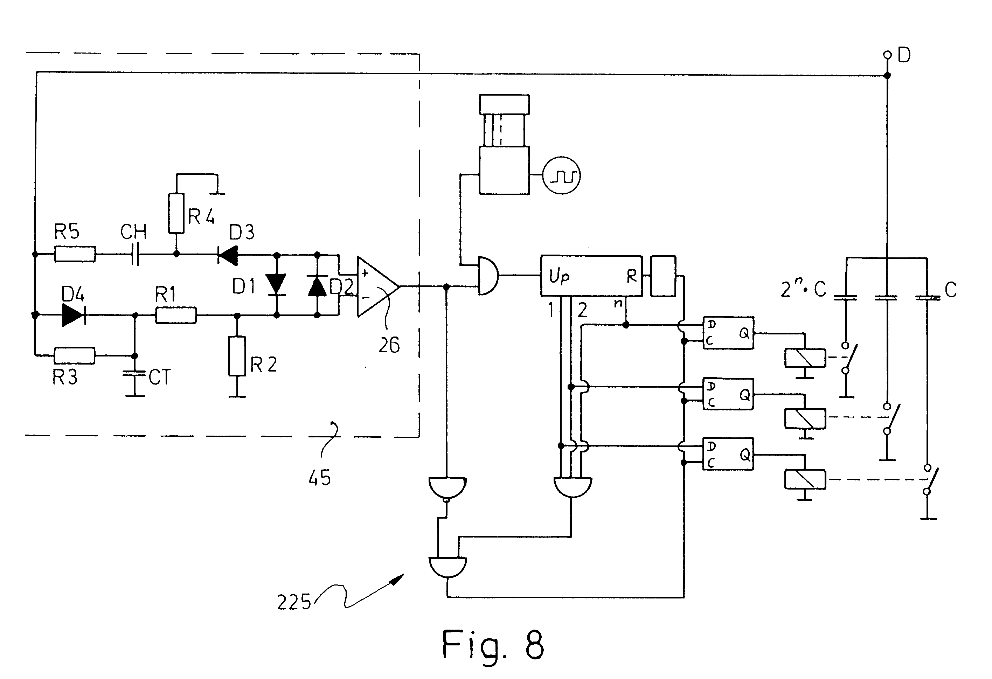

Im folgenden wird eine weitere bevorzugte Ausführungsform der Erfindung anhand der Fig. 7 und 8 näher beschrieben. Bei dieser Ausführungsform der Erfindung ist eine abgeänderte Abgleichvorrichtung 225 vorgesehen, die ebenfalls (wie in Fig. 2) parallel zum Kondensator $C_o$ geschaltet ist, jedoch keine weiteren Eingänge besitzt. Bei dieser Schaltung wird die Spannung am Kondensator $C_o$ über eine Diode $D_4$ auf einen Kondensator CT geführt, wobei hier eine entsprechende Polung des Netzteils 21 vorausgesetzt ist. Sobald also der Kondensator $C_o$ aufgeladen wird, steht die gleiche Spannung (abzüglich der Durchlaßspannung der Diode $D_4$) am Kondensator CT an. Entlädt sich der Kondensator $C_o$, so ist der Punkt D auf niedrigerem Potential als der entsprechende Anschlußpunkt des Kondensators CT, so daß die Diode $D_4$ sperrt. Parallel zur Diode $D_4$ ist ein hochohmiger Widerstand $R_3$ geschaltet, über den sich der Kondensator CT sehr langsam entladen kann, was notwendig ist, um eine eventuelle Umänderung der Netzspannung wirksam werden lassen zu können.

Dem Kondensator CT nachgeschaltet ist wieder der Spannungsteiler $R_1$, $R_2$, dessen Ausgangsspannung auf dem invertierenden Eingang des Operationsverstärkers 26 sitzt.

Derselbe Potentialpunkt D wird über einen Schutz-Widerstand $R_5$ auf einen Hochpass, bestehend aus Kondensator CH und Widerstand $R_4$, geführt. Der Ausgang des Hochpasses ist auf die Kathode einer Diode $D_3$ geführt, deren Anode auf dem nicht-invertierenden Eingang des Operationsverstärkers 26 liegt. Wenn nun der Kondensator einmal aufgeladen ist, so bleibt auch während der Entladung (bei entsprechender Dimensionierung des Widerstandes $R_3$) die Maximalspannung (entsprechend der Netzteil-Ausgangsspannung) am Kondensator CT erhalten, so daß am invertierenden Eingang des Operationsverstärkers 26 die eingangs beschriebene Schwellenspannung $U_s$ liegt. Der Abfall der Spannung am Kondensator $C_o$ wird allerdings über den Hochpass $CH/R_4$ und die Diode $D_3$ dem nicht invertierenden Eingang des Operationsverstärkers 26 mitgeteilt, so daß an diesem Eingang wieder die in Fig. 4A gezeigte Impulsform der Kondensator-Entladekurve anliegt.

Weiterhin ist es möglich, anstelle eines Binärzählers zum Beispiel einen Dezimalzähler zu verwenden, wobei dann die Kapazitätswerte entsprechend den Dezimalstellen zu wählen sind. Hierbei ist es (sowohl bei Binär- als auch bei Dezimaleinstellung) von Vorteil, wenn bei im wesentlichen bekanntem Kapazitätswert von $C_o$ für eine bekannte Soll-Zeitkonstante die Werte der Kompensationskondensatoren bzw. Widerstände nur einem Feinabgleich entsprechend gewählt sind, was die Anzahl der notwendigen Kondensatoren und Relais erheblich verringern kann.

Alle beschriebenen Einzelmerkmale werden für sich allein als erfindungswesentlich beansprucht, ebenso deren Kombination, was insbesondere für die Kombination von Möglichkeiten zur Veränderung der Zeitkonstanten gilt (Parallelschaltung von Kapazitäten, Reihenschaltung von Widerständen). Gleiches gilt für die im Ausführungsbeispiel nicht gezeigte direkte Konstanthaltung der Entlade-Kurvenform.

**Patentansprüche**

1. Verfahren zur Behandlung von Stoffen und/oder Mikro-Organismen mit Elektroimpulsen in einem kontinuierlich arbeitenden System, wobei man die zu behandelnden Stoffe Impulsen definierter Feldstärke aussetzt, deren zeitlicher Verlauf durch Entladung mindestens eines Speicherkondensators und der Impedanz einer Entladungsstrecke bestimmt wird, **dadurch gekennzeichnet**, daß man einen Optimalwert der für die Entladung bestimmenden Soll-Zeitkonstante ($\tau_s$) bestimmt, bei welchem die gewünschte Wirkung der Elektroimpulse auf den behandelten Stoff bzw. der entsprechende Parameter maximal ist, daß man die Kapazität und/oder Impedanz auf die Soll-Zeitkonstante ($\tau_s$) einstellt und daß man die Zeitkonstante unabhängig von der Impedanz des Systems (Leitfähigkeit des Stoffes) konstant hält.

2. Verfahren nach Anspruch 1, dadurch gekennzeichnet, daß man den zu maximierenden Parameter kontinuierlich am Systemausgang mißt und die Zeitkonstante ($\tau_x$) solange abändert, bis der Parameter seinen Maximalwert erreicht und den dann vorliegenden Wert der Zeitkonstante ($\tau_x$) als Soll-Zeitkonstante ($\tau_x = \tau_s$) speichert.

3. Verfahren nach einem der vorhergehenden Ansprüche, dadurch gekennzeichnet, daß man zur Einstellung der Zeitkonstante ($\tau_x$) die Kapazität der Speicherkondensatoren verändert, insbesondere durch Parallelschaltung definierter Anzahlen von Kondensatoren.

4. Verfahren nach einem der vorhergehenden Ansprüche, dadurch gekennzeichnet, daß man die Zeitkonstante ($\tau_x$) durch Einstellung des Widerstands eines mit dem Kondensator und der Entladungsstrecke in Reihe geschalteten Vorwiderstands (-netzwerkes) einstellt.

5. Verfahren nach einem der vorhergehenden Ansprüche, dadurch gekennzeichnet, daß man die Zeitkonstante ($\tau_x$) einstellt, indem man die tatsächliche Zeitkonstante ($\tau_n$) eines Impulses mißt, mit der Soll-Zeitkonstante ($\tau_s$) vergleicht, aus der Differenz ($\tau_s - \tau_n$) einen Korrekturwert ($\tau_k$) errechnet und die Kapazität des Kondensators und/oder den Wert des Vorwiderstands

entsprechend dem Korrekturwert ($\tau_k$) verändert, bevor die nächste Kondensatorentladung erfolgt.

6. Verfahren nach Anspruch 5, dadurch gekennzeichnet, daß man den Korrekturwert ($\tau_k$) aus der Differenz zwischen Soll-Zeitkonstante ($\tau_s$) und dem Mittelwert der tatsächlichen Zeitkonstanten über eine definierte Anzahl von Impulsen bildet.

7. Vorrichtung zur Behandlung von Stoffen und/Mikroorganismen mit Elektroimpulsen in einem kontinuierlich arbeitenden System, mit mindestens einem Kondensator ($C_o$), der über eine Gleichspannung (Netzteil 21) aufladbar und über einen gesteuerten (Impulssteuerung 20) Schalter (22) mit Elektroden (11, 12) zur Entladung verbindbar ist,
dadurch gekennzeichnet, daß eine Abgleichvorrichtung (25) vorgesehen ist, die mindestens während des Entladevorganges mit dem Entladungsstromkreis ($C_o$, 11, 12, 22) zum Abtasten des Entladungsverlaufes verbunden ist, die Mittel (45, 46) zum Vergleichen des Entladevorganges mit einem vorbestimmten Wert ($\tau_s$) und Mittel (47) zum Angleichen des Entladevorganges an den vorbestimmten Wert ($\tau_s$) aufweist.

8. Vorrichtung nach Anspruch 7, dadurch gekennzeichnet, daß die Abgleichvorrichtung (25) umfaßt
   a) einen (Analog-)Komparator (45), der die Spannung am Kondensator ($C_o$) während des Entladens mit einer Schwelle ($U_s$) vergleicht, die einem definierten Bruchteil (1/e) der Kondensatorspannung zu Beginn der Entladung entspricht,
   b) eine Meßvorrichtung (46) zum Feststellen des Zeitraumes ($\tau_x$) zwischen Entladungsbeginn und Unterschreiten der Schwelle ($U_s$),
   c) eine Kompensationsschaltung (47) zum Verändern der Kapazität und/oder Impedanz des Entladestromkreises entsprechend der Differenz zwischen vorbestimmtem Wert ($\tau_s$) und gemessenem Wert ($\tau_x$).

9. Vorrichtung nach Anspruch 8, dadurch gekennzeichnet, daß der Komparator (45) eingangsseitig einerseits über einen Spannungsteiler ($R_1$, $R_2$) mit der Lade-Gleichspannung (Netzteil 21), andererseits mit einer Elektrode (12) verbunden ist.

10. Vorrichtung nach Anspruch 8, dadurch gekennzeichnet, daß der Komparator (45) eingangsseitig einerseits über einen schnell aufladenden ($D_4$) und langsam ($R_3$) entladenden Spannungsspeicher (CT) und eine Spannungsteilerschaltung ($R_1$, $R_2$) mit dem einen Pol (D) des Kondensators ($C_o$), andererseits über einen Hochpass ($C_4$, $R_4$) mit nachgeschaltetem Gleichrichter ($D_3$) mit dem Pol (D) des Kondensators ($C_o$) verbunden ist.

11. Vorrichtung nach einem der Ansprüche 8 bis 10, dadurch gekennzeichnet, daß die Meßvorrichtung (46) einen Zähler (31) umfaßt, dessen Zählereingang (Up) über ein UND-Gatter (27) mit einem Taktgenerator (34) verbunden ist, wobei das UND-Gatter (27) mit seinem zweiten Eingang mit dem Ausgang der Komparatorschaltung (45) verbunden ist, so daß innerhalb des Zeitraumes ($\tau_x$) zwischen Entladungsbeginn und Unterschreiten der Schwelle ($U_s$) Taktimpulse zählbar sind.

12. Vorrichtung nach Anspruch 11, dadurch gekennzeichnet, daß zwischen Taktgenerator (34) und UND-Gatter (27) ein Frequenzteiler (32) angeordnet ist, dessen Teilverhältnis über Einstellmittel (33) einstellbar ist, die vorzugsweise eine Recheneinheit (41) umfassen, der Ausgangssignale eines zur Messung eines Parameters der zu behandelnden Stoffe/Mikroorganismen dienenden Meßfühlers (44) zuführbar sind, wobei die Recheneinheit (41) über eine Eingabetastatur (42) einstellbar ist.

13. Vorrichtung nach einem der Ansprüche 8 bis 12, dadurch gekennzeichnet, daß die Kompensationsschaltung (47) eine Vielzahl von Kompensations-Kondensatoren (C) und/oder Kompensations-Widerständen (R) umfaßt, die parallel/in Serie zum Kondensator ($C_o$) schaltbar sind, wobei vorzugsweise die Kondensatoren (C) bzw. Widerstände (R) ihrem Wert nach in ihrer Gesamtwirkung kleiner sind als der Kondensator ($C_o$) bzw. die Impedanz des Entladestromkreises.

14. Vorrichtung nach den Ansprüchen 11 und 13, dadurch gekennzeichnet, daß der Zähler (31) ein Binärzähler ist, dessen Inhalt über Speicherschaltungen (36) mit Schaltern (37) zu deren Betätigung verbunden ist, um die Kompensations-Kondensatoren (C) bzw. -Widerstände (R) ein- und auszuschalten, und daß die Kompensations-Kondensatoren (C) bzw. -Widerstände (R) den Binärzählerstellen entsprechende Werte ($2^n$ x C bzw. R) aufweisen.

## Claims

1. Method for treating substances and/or microorganisms with electric pulses in a continuously operating system, the substances to be treated being exposed to pulses of defined field strength, the variation with time of which is determined by discharging at least one storage capacitor and by the impedance of a discharge path, characterised in that an optimum value of the nominal time constant ($\tau_s$) determining the discharge is determined at which the desired effect of the electric pulses on the treated substance or the corresponding parameter is at a maximum, that the capacitance and/or impedance is set to the nominal time constant ($\tau_s$) and that the time constant is kept constant independently of the impedance of the system (conductivity of the substance).

2. Method according to Claim 1, characterised in that the parameter to be maximised is continuously measured at the system output and the time constant ($\tau_x$) is changed until the parameter reaches its maximum value and the value of the time constant ($\tau_x$) then present is stored as nominal time constant ($\tau_x = \tau_s$).

3. Method according to one of the preceding claims, characterised in that the capacitance of the storage capacitors is changed for setting the time constant ($\tau_x$) connecting defined numbers of capacitors in parallel.

4. Method according to one of the preceding claims, characterised in that the time constant ($\tau_x$) is set by setting the resistance of a series resistance (network) connected in series with the capacitor and the discharge path.

5. Method according to one of the preceding claims, characterised in that the time constant ($\tau_x$) is set by measuring the actual time constant ($\tau_n$) of one pulse, comparing it with the nominal time constant ($\tau_s$), calculating a correction value ($\tau_k$) from the difference ($\tau_s - \tau_n$) and changing the capacitance of the capacitor and/or the value of the series resistance in accordance with the correction value ($\tau_k$) before the next capacitor discharge occurs.

6. Method according to Claim 5, characterised in that the correction value ($\tau_k$) is formed from the difference between the nominal time constant ($\tau_s$) and the mean value of the actual time constants over a defined number of pulses.

7. Device for treating substances and/or microorganisms with electric pulses in a continuously operating system, with at least one capacitor ($C_o$) which can be charged via a direct voltage (power supply 21) and can be connected via a controlled (pulse control 20) switch (22) to electrodes (11, 12) for discharging, characterised in that a calibration device (25) is provided which is connected to the discharge circuit ($C_o$, 11, 12, 22), at least during the discharge process, for sampling the progression of discharge, which exhibits means (45, 46) for comparing the discharge process with a predetermined value ($\tau_s$) and means (47) for matching the discharge process to the predetermined value ($\tau_s$).

8. Device according to Claim 7, characterised in that the calibration device (25) comprises
   a) an (analog) comparator (45) which comprises the voltage across the capacitor ($C_0$) during the discharging with a threshold ($U_s$) which corresponds to a defined fraction (1/e) of the capacitor voltage at the beginning of the discharge,
   b) a measuring device (46) for determining the period of time ($\tau_x$) between the beginning of discharge and when the voltage drops below the threshold ($U_s$).
   c) a compensation circuit (47) for changing the capacitance and/or impedance of the discharge circuit in accordance with the difference between the predetermined value ($\tau_s$) and the measured value ($\tau_x$).

9. Device according to Claim 8, characterised in that the input of the comparator (45) is connected, on the one hand, to the charging direct voltage (power supply 21) via a voltage divider ($R_1$, $R_2$) and, on the other hand, to an electrode (12).

10. Device according to Claim 8, characterised in that the input of the comparator (45) is connected, on the one hand, via a rapidly charging ($D_4$) and slowly ($R_3$) discharging voltage store (CT) and a voltage divider circuit ($R_1$, $R_2$) to one pole (D) of the capacitor ($C_o$) and, on the other hand, via a high-path filter ($C_4$, $R_4$) with subsequent rectifier ($D_3$) to the pole (D) of the capacitor ($C_o$).

11. Device according to one of Claims 8 to 10, characterised in that the measuring device (46) comprises a counter (31), the counter input ($U_p$) of which is connected via an AND gate (27) to a clock generator (34), the second input of the AND gate (27) being connected to the output of the comparator circuit (45) so that clock pulses can be counted within the period of time ($\tau_x$) between the beginning of discharge and when the voltage drops below the threshold ($U_s$).

12. Device according to Claim 11, characterised in that a frequency divider (32), the dividing ratio of which can be adjusted via adjusting means (33), is arranged between clock generator (34) and AND gate (27), which adjusting means preferably comprise an arithmetic unit (41) which can be supplied with output signals of a measuring sensor (44) used for measuring a parameter of the substances/microorganisms to be treated, the arithmetic unit (41) being adjustable via an input keyboard (42).

13. Device according to one of Claims 8 to 12, characterised in that the compensation circuit (47) comprises a plurality of compensation capacitors (C) and/or compensation resistors (R) which can be connected in parallel/in series with the capacitor ($C_o$) in which the total effect of the value of the capacitors (C) or resistors (R) is preferably less than that of the capacitor ($C_o$) or of the impedance of the discharge circuit.

14. Device according to Claims 11 and 13, characterised in that the counter (31) is a binary counter the content of which is connected via storage circuits (36) to switches (37) for actuating these circuits in order to connect and disconnect the compensation capacitors (C) or resistors (R), and that the compensation capacitors (C) or resistors (R) exhibit values ($2^n$ x C or R) corresponding to the binary counter digits.

**Revendications**

1. Procédé de traitement de matières et/ou de microorganismes avec des impulsions électriques dans un système fonctionnant en continu, dans lequel on soumet les matières à traiter à des impulsions d'intensité de champ définie dont l'allure dans le temps est déterminée par la décharge d'au moins un condensateur réservoir et l'impédance d'une voie de décharge, caractérisé par le fait qu'on détermine une valeur optimale de la constante de temps prescrite déterminante pour la décharge ($\tau_s$) avec laquelle l'effet désiré des impulsions électriques sur la matière traitée ou le paramètre correspondant est maximal, qu'on règle la capacité et/ou l'impédance à la constante de temps prescrite ($\tau_s$) et qu'on maintient la constante de temps constante indépendamment de l'impédance du système (conductivité de la matière).

2. Procédé selon la revendication 1, caractérisé par le fait qu'on mesure de façon continue, à la sortie du système, le paramètre à rendre maximal et modifie la constante de temps ($\tau_s$) jusqu'à ce que le paramètre atteigne sa valeur maximale et met en mémoire, comme constante de temps prescrite ($\tau_x = \tau_s$), la valeur alors présente de la constante de temps ($\tau_s$).

3. Procédé selon l'une des revendications précédentes, caractérisé par le fait que, pour régler la constante de temps ($\tau_x$), on modifie la capacité des condensateurs, en particulier par couplage en parallèle de nombres définis de condensateurs.

4. Procédé selon l'une des revendications précédentes, caractérisé par le fait qu'on règle la constante de temps ($\tau_x$) en réglant la résistance d'une résistance additionnelle (d'un réseau de résistances additionnelles) monté(e) en série avec le condensateur et la voie de décharge.

5. Procédé selon l'une des revendications précédentes, caractérisé par le fait qu'on règle la constante de temps ($\tau_x$) en mesurant la constante de temps effective ($\tau_n$) d'une impulsion, en la comparant à la constante de temps prescrite ($\tau_s$), en calculant, à partir de la différence ($\tau_s - \tau_n$), une valeur de correction ($\tau_k$) et en modifiant la capacité du condensateur et/ou la valeur de la résistance additionnelle d'après cette valeur de correction ($\tau_k$) avant qu'ait lieu la décharge de condensateur suivante.

6. Procédé selon la revendication 5, caractérisé par le fait qu'on établit la valeur de correction ($\tau_k$) à partir de la différence entre la constante de temps prescrite ($\tau_s$) et la moyenne des constantes de temps réelles sur un nombre défini d'impulsions.

7. Dispositif de traitement de matières et/ou de microorganismes avec des impulsions électriques dans un système fonctionnant en continu, comportant au moins un condensateur ($C_o$) qui peut être chargé au moyen d'une tension continue (bloc d'alimentation 21) et être relié pour la décharge à des électrodes (11, 12) par l'intermédiaire d'un interrupteur (22) commandé (commande d'impulsions 20), caractérisé par le fait qu'il est prévu un dispositif d'alignement (25) qui, au moins pendant la décharge, est relié au circuit de décharge ($C_o$, 11, 12, 22) pour l'analyse de la décharge, et présente des moyens (45, 46) de comparaison de la décharge à une valeur prédéterminée ($\tau_s$) et des moyens (47) d'adaptation de la décharge à cette valeur prédéterminée ($\tau_s$).

8. Dispositif selon la revendication 7, caractérisé par le fait que le dispositif d'alignement (25) comprend

a) un comparateur (analogique) (45) qui compare la tension aux bornes du condensateur ($C_o$) pendant la décharge à un seuil ($U_s$) qui correspond à une fraction définie ($1/e$) de la tension aux bornes du condensateur au début de la décharge.

b) un dispositif de mesure (46) pour la mesure du laps de temps ($\tau_x$) entre le début de la décharge et le franchissement du seuil ($U_s$) vers le bas,

c) un circuit de compensation (47) pour la modification de la capacité et/ou de l'impédance du circuit de décharge d'après la différence entre la valeur prédéterminée ($\tau_s$) et la valeur mesurée ($\tau_x$).

9. Dispositif selon la revendication 8, caractérisé par le fait que le comparateur (45) est relié du côté entrée, d'une part, par l'intermédiaire d'un diviseur de tension ($R_1$, $R_2$), à la tension continue de charge (bloc d'alimentation 21), d'autre part à une électrode (12).

10. Dispositif selon la revendication 8, caractérisé par le fait que le comparateur (45) est relié du côté entrée, d'une part, par l'intermédiaire d'un accumulateur de tension (CT) se chargeant rapidement ($D_4$) et se déchargeant lentement ($R_3$) et d'un circuit diviseur de tension ($R_1$, $R_2$), à un pôle (D) du condensateur ($C_o$), d'autre part, par l'intermédiaire d'un filtre passe-haut ($C_4$, $R_4$) avec redresseur ($D_3$) monté en aval, au pôle (D) du condensateur ($C_o$).

11. Dispositif selon l'une des revendications 8 à 10, caractérisé par le fait que le dispositif de mesure (46) comprend un compteur (31) dont l'entrée de comptage (Up) est reliée, par l'intermédiaire d'une porte ET (27), à un générateur d'horloge (34), la deuxième entrée de la porte ET (27) étant reliée à la sortie du circuit comparateur (45), de sorte que, dans le laps de temps ($\tau_x$) entre le début de la décharge et le franchissement du seuil ($U_s$) vers le bas, peuvent être comptées des impulsions d'horloge.

12. Dispositif selon la revendication 11, caractérisé par le fait qu'entre le générateur d'horloge (34) et la porte ET (27) est monté un diviseur de fréquence (32) dont le rapport de division est réglable par des moyens de réglage (33) qui comprennent de préférence un organe de calcul (41) auquel peuvent être envoyés des signaux de sortie d'un capteur de mesure (44) servant à la mesure d'un paramètre des matières et/ou micro-organismes à traiter, cet organe de calcul (41) étant réglable par un clavier (42).

13. Dispositif selon l'une des revendications 8 à 12, caractérisé par le fait que le circuit de compensation (47) comprend un grand nombre de condensateurs de compensation (C) et/ou de résistances de compensation (R) qui peuvent être couplés en parallèle/en série avec le condensateur ($C_o$), ces condensateurs (C) et résistances (R) étant de préférence, quant à leur effet total, inférieurs en valeur, respectivement, au condensateur ($C_o$) et à l'impédance du circuit de décharge.

14. Dispositif selon les revendications 11 et 13, caractérisé par le fait que le compteur (31) est un compteur binaire dont le contenu est relié par l'intermédiaire de circuits de mémoire (36) à des interrupteurs (37) pour l'actionnement de ceux-ci pour la mise en circuit et hors circuit des condensateurs (C) et des résistances (R) de compensation, et que les condensateurs (C) et les résistances (R) de compensation ont des valeurs ($2^n \times C$ ou $2^n \times R$) correspondant aux chiffres du compteur binaire.

Fig. 1

Fig. 2

Fig. 3

EP 0 283 700 B1

Fig. 4

Fig. 5

Fig. 6

Fig. 7

Fig. 8

EP 0 283 700 B1